# EUROPEAN PATENT APPLICATION

(11) **EP 4 501 390 A1**
(43) Date of publication of application: **05.02.2025**
(21) Application number: 23775011.2
(22) Date of filing: 23.03.2023
(51) Int. Cl.: A61M 27/00, A61F 13/00

(54) **WOUND TREATMENT DEVICE AND WOUND COVERING USED THEREIN**

(30) Priority: 25.03.2022 JP 2022050527
(71) Applicant: OSAKA UNIVERSITY, Suita-shi Osaka 565-0871 (JP)
(72) Inventor: YAGI, Masakazu, Suita-shi, Osaka 565-0871 (JP); SAWA, Yoshiki, Suita-shi, Osaka 565-0871 (JP); MIYAGAWA, Shigeru, Suita-shi, Osaka 565-0871 (JP); YAMADA, Shohei, Suita-shi, Osaka 565-0871 (JP); MASUDA, Hirotada, Suita-shi, Osaka 565-0871 (JP); MIYAKE, Keisuke, Suita-shi, Osaka 565-0871 (JP); KOSHIHARA, Takayuki, Kobe-shi, Hyogo 651-0073 (JP)
(74) Representative: Mewburn Ellis LLP
(86) International application number: PCT/JP2023/011470
(87) International publication number: WO 2023/182417

(57) **Abstract**

The present invention provides a wound treatment device with which there is no increase in infection or delay in healing due to, inter alia, an irrigation liquid leakage or foam adjustment, embedment, or replacement, and that can effectively protect the surroundings of a wound and can be easily handled without requiring skill. The present invention also provides a wound covering used therein. The present invention provides a wound treatment device characterized by comprising a wound covering constituted from a flexible laminate including a porous member and a watertight film member laminated on the porous member, a negative pressure supply pump for supplying negative pressure to the porous member side, an irrigation fluid pump for supplying an irrigation fluid to the porous member side and discharging the irrigation fluid from the porous member side, a canister for intaking the discharged irrigation fluid, and a control unit for controlling the negative pressure supply pump and the irrigation fluid pump, the wound treatment device performing negative pressure wound therapy and irrigation on the wound covered on the porous member side by the wound covering.

## Description

### TECHNICAL FIELD

The present invention relates to a wound treatment device for treating a wound and to a wound portion covering body used therein.

### BACKGRAOUND ART

With the worldwide spread of diabetes and arteriosclerosis obliterans, the incidence of foot ulcers in particular is increasing. The foot ulcers are easily exposed to external pressure and friction and are difficult to heal. Further, infected ulcers are extremely difficult to treat and tend to spread to deeper tissues such as bones, leading to major amputations of the lower limbs and threatening the life of patient.

Negative pressure wound therapy is used to treat wounds such as ulcers as described above, in which, first, a healthcare provider uses scissors or the like to cut a piece of foam (a sponge-like material) to the shape and dimensions of the portion of wound (wound portion), embeds the cut foam in the wound portion, places a film on top thereof, makes a hole in one place and applies negative pressure to promote wound healing. The wound portion is then intermittently filled with saline or the like to wash it out so as not to increase infection at the wound portion.

Here, the afore-mentioned negative pressure wound therapy has the following disadvantages (1) to (3).
(1) Because the foam is embedded in the wound portion, an airtight space may be created, which may increase the risk of infection, and when the foam is replaced, granulation tissue may get in and hinder healing (problems with embedding and replacing the foam).
(2) Protection is limited to the wound portion itself, and the surrounding area is not protected from external pressure or friction, which may lead to the formation of new wounds (problems with protecting the area around the wound portion).
(3) Skill is required to cut and adjust the foam to fit the shape of the wound portion, and each treatment takes 30 to 50 minutes (problems of skill and time).

In response to this, for example, Patent Document 1 (Japanese Patent Publication No. 2017-527336) discloses and proposes various combination fluid instillation and negative pressure dressings as devices used in negative pressure wound therapy.

### CITATION LIST

### Patent Document

Patent Document 1: Japanese Patent Publication No. 2017-527336

### SUMMARY OF THE INVENTION

### Problem to be solved by the invention

However, the combined fluid instillation and negative pressure dressing, which is the device used for the negative pressure wound therapy described in Patent Document 1, has a complicated structure and cannot sufficiently alleviate the above problems (1) to (3), and further, there is still a risk of scattering the irrigation liquid to the surrounding area, which may increase infection.

Therefore, the object of the present invention is to provide a wound treatment device with which there is no increase in infection or delay in healing due to, inter alia, an irrigation liquid leakage or foam adjustment, embedment, or replacement, and that can effectively protect the surroundings of a wound portion and can be easily handled without requiring skill, and to provide a wound portion covering body used therein, and as a result of repeated prototypes and intensive research, the inventors have finally completed the present invention.

### MEANS TO SOLVE THE PROBLEMS

Namely, the present invention provides a wound treatment device characterized by comprising:
a wound portion covering body constituted from a flexible laminate including a porous member and a watertight cover member laminated on the porous member,
a negative pressure supply pump for supplying negative pressure to the porous member side,
an irrigation fluid pump for supplying an irrigation fluid to the porous member side and discharging the irrigation fluid from the porous member side,
a canister for intaking the discharged irrigation fluid, and
a control unit for controlling the negative pressure supply pump and the irrigation fluid pump,
wherein subjecting the wound portion covered on the porous member side by the wound portion covering body to negative pressure wound therapy and irrigation.

It is preferable that,
in the wound treatment device of the present invention described above,
the porous member has a number of convex portions on the surface opposite the cover member.

Further, it is preferable that, in the wound treatment device of the present invention described above,
the wound portion covering body is pad-shaped, approximately cylindrical, approximately bag-shaped or approximately shoe-shaped.

Further, in the wound treatment device of the present invention described above, the irrigation fluid may be any fluid capable of irrigating, sterilizing, and the like the wound portion, and may be, for example, a liquid such as an irrigation solution, or may be, for example, a bactericidal or healing-promoting gas. The irrigation fluid, in the case of a liquid irrigation liquid, it is preferable to contain an aqueous solution of peroxynitric acid.

Further, it is preferable that,
in the wound treatment device of the present invention described above,
the canister is equipped with a sensing unit that measures the number of bacteria in the irrigation fluid.

Further, it is preferable that, in the wound treatment device of the present invention described above,
the control unit is capable of adjusting the number of washings depending on the number of bacteria.

Furthermore, the present invention also provides a wound portion covering body that is suitably used in the above-mentioned wound treatment device of the present invention. The wound portion covering body of the present invention is characterized by:
constituted from a flexible laminate including a porous member and a watertight cover member laminated on the porous member, and
used for subjecting the wound portion covered on the porous member side to negative pressure wound therapy and irrigation.

It is preferable that,
in the wound portion covering body of the present invention described above,
the porous member has a number of convex portions on the surface opposite the cover member.

It is preferable that,
in the wound portion covering body of the present invention described above,
the shape is pad-shaped, approximately cylindrical, approximately bag-shaped or approximately shoe-shaped.

### EFFECT OF THE INVENTION

According to the present invention, it is possible to obtain a wound treatment device with which there is no increase in infection or delay in healing due to, inter alia, an irrigation liquid leakage or foam adjustment, embedment, or replacement, and that can effectively protect the surroundings of a wound portion to suppress complications and can be easily handled without requiring skill, and to provide a wound portion covering body used therein. That is, according to the wound treatment device and wound portion covering body used therein of the present invention, it is possible to realize infection control, early healing, prevention of complications, and shortened time and simplification, which were difficult to achieve with conventional wound treatments.

### BRIEF DESCRIPTION OF THE DRAWINGS

FIG. 1 is a schematic diagram conceptually showing the configuration of the wound treatment device 1 according to one embodiment of the present invention.
FIG. 2 is a schematic diagram for explaining the function of the wound portion covering body in the wound treatment device 1 shown in FIG. 1.
FIG. 3 is a schematic diagram for explaining the function of the wound portion covering body in the wound treatment device 1 shown in FIG. 1.
FIG. 4 is a schematic diagram conceptually showing the wound treatment device 1A according to one modified embodiment of the present invention.
FIG. 5 is another schematic diagram conceptually showing the wound treatment device 1A shown in FIG. 4.
FIG. 6 is a schematic diagram conceptually showing the wound treatment device 1B according to another modified embodiment of the present invention.

### MODE FOR CARRYING OUT THE INVENTION

Hereinafter, an apparatus according to a representative embodiment of the present invention will be described in detail with reference to the drawings, but the present invention is not limited thereto. However, since the drawings are intended to conceptually explain the present invention, there are the cases where the dimensions, ratios, or numbers may be exaggerated or simplified in order to facilitate understanding.

### <Configuration of the wound treatment device>

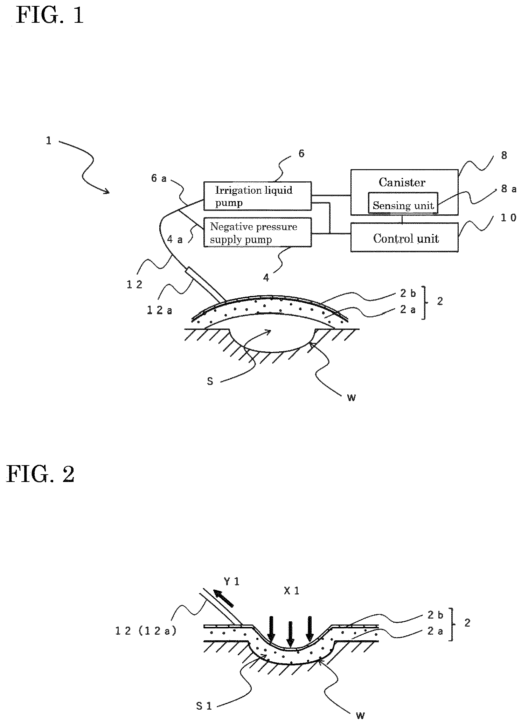

As shown in FIG. 1, the wound treatment device 1 of one embodiment of the present invention includes a wound portion covering body 2, a negative pressure supply pump 4, an irrigation fluid pump 6, a canister 8, and a control unit 10, and is capable of performing negative pressure wound therapy and irrigating on the wound portion by using the wound portion covering body 2. In this embodiment, an irrigation liquid is used as the irrigation fluid, and therefore an irrigation liquid pump is used as the irrigation fluid pump.

The wound portion covering body 2 of this embodiment is a pad-shaped body that covers the wound portion including the wound and its surrounding area, and is constituted from a flexible laminate including a porous member 2a and a watertight cover member 2b laminated to the porous member 2a. The term "flexible" as used herein means that when a certain pressure is applied, the material can be distorted and deformed upwardly and convexly (approximately dome-shaped) or downwardly and concavely (approximately dish-shaped).

The porous member 2a is a so-called sponge-like foam (foamed material) having a three-dimensional network structure, and various materials can be used as long as imparting flexibility to the wound portion laminating body 2, and, for example, resinous materials such as polyurethane, polyester, and melamine can be used. Further, the porous member 2a may have a certain degree of water absorption, but may also have hydrophobicity so as not to reduce handrability by retaining excess exudate from the wound portion and irrigation fluid.

Although not shown in FIG. 1, the foam constituting the porous member 2a preferably has a plurality of convex portions (such as mountain-shaped protrusions) on the surface facing the wound portion W (i.e., the surface opposite the cover member 2b). The presence of such a convex portion allows the porous member 2a to more reliably contact and cover the wound at the wound portion, which contributes to early healing. In particular, it is preferable that a plurality of the convex portions is provided in a regular array.

Note, the wound portion covering body 2 can have various shapes as long as being pad-shaped, and may be, for example, approximately rectangular or approximately circular. Further, the thickness of the porous member 2a can be appropriately selected within a range where the effects of the present invention are not impaired, and may be adjusted appropriately depending on, for example, the location and extent of the wound portion, while the height of the convex portion may be, for example, around 0.2 mm to 4 cm.

Next, the cover member 2b is laminated to the porous member 2a through adhesion by using an adhesive, heat fusion, or the like, and can also be called a backing layer, and has watertightness and has a certain degree of elasticity to impart flexibility to the wound portion laminating body 2. As such a cover member 2b, various materials can be used as long as imparting flexibility and watertightness to the wound portion laminating body 2, and, for example, may be made of materials such as polyurethane, latex, nitrile, silicone resin, and various thermoplastic resins. The cover member may be in the form of a film or a sheet.

The negative pressure supply pump 4 supplies a negative pressure to the porous member 2a side of the wound portion covering body 2 via a negative pressure supply pipe 4a and a pipe 12. Thereby, as will be described later with reference to FIG. 2, the negative pressure can be supplied between the wound portion covering body 2 and the wound portion W, and the wound portion covering body 2 can be brought into close contact with the wound portion W on the porous member 2a side.

As the negative pressure supply pump 4, any conventionally known pump can be used as long as the effects of the present invention are not impaired.

Further, the irrigation liquid pump 6 supplies irrigation liquid such as saline or a peroxynitrite solution to the porous member 2a side of the wound portion covering body 2 via the irrigation liquid tube 6a and tube 12, filling the space between the porous member 2a and the wound portion W with the irrigation liquid, and performing irrigation (infection treatment) to remove bacteria from the surface of the wound portion W by immersing in the liquid. In other words, a positive pressure is supplied between the wound portion covering body 2 and the wound portion W. Further, the irrigation liquid pump 6 discharges the irrigation liquid between the porous member 2a and the wound portion W to the outside of the wound portion covering body 2.

As the irrigation liquid pump 6, any conventionally known pump may be used as long as the effects of the present invention are not impaired. Although not shown, the irrigation liquid may be supplied by connecting a separate irrigation liquid pack or the like to the irrigation liquid pump 6.

The canister 8 is a container that collects the irrigation liquid discharged after irrigating the wound portion W. As such a canister, any conventionally known canister can be used as long as the effects of the present invention are not impaired. For example, it is also possible to use a canister that may contain a gelling agent to solidify the discharged irrigation liquid so that it does not dissipate.

Further, it is also preferable that the canister 8 is equipped with a sensing unit 8a that measures the number of bacteria in the discharged irrigation liquid, and furthermore, it is preferable that the number of irrigations by using the irrigation liquid pump 6 can be adjusted according to the measured number of bacteria by the control unit 10 described later. As a canister having such a function, any conventionally known canister may be used.

The negative pressure supply pump 4, the irrigation liquid pump 6 and the canister 8 having the sensing unit 8a can be controlled by a control unit 10. The control unit 10 includes a liquid crystal display unit or an LED display unit, and may be of the button type or touch panel type, and may be realized by conventionally known technology.

### <How to use the wound treatment device>

Next, the way how to use the wound treatment device 1 of this embodiment will be briefly explained. First, as shown in FIG. 1, the wound portion W is covered with the mechanism in which the pad-shaped wound portion covering body 2 is adhered to the cover member, and the like. In some cases, although not shown, the pad-shaped wound portion covering body 2 and the cover member may be separated before use, and may be fixed by adhering their peripheries together when covering. At this time, a space exists between the wound portion W and the porous member 2a of the wound portion covering body 2.

Next, when the power is turned on by the control unit 10, the negative pressure treatment mode is automatically or manually selected, and the negative pressure supply pump 4 is activated to supply a negative pressure X1 between the wound portion covering body 2 and the wound portion W, as shown in FIG. 2, and by exhausting air in the direction Y1, the wound portion covering body 2 is bent and deformed downwardly concave (approximately dish-shaped) to bring the porous member 2a into close contact with the wound portion W. At this time, the space S in FIG. 1 is filled with the porous member 2a as shown by S1 in FIG. 2. Thereby, it is possible to carry out the negative pressure therapy that has the effects of improvement of microcirculation, removal of excess exudate, improvement of tissue edema, and shrinkage of the wound portion W.

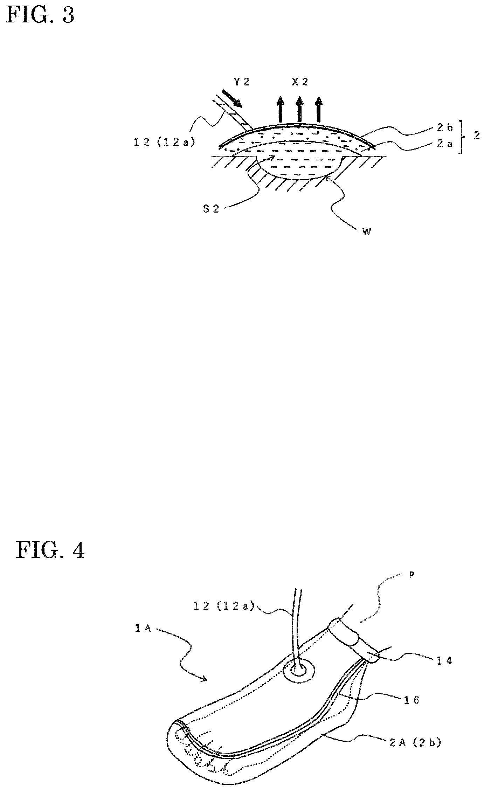

Next, the irrigation liquid pump 6 is operated by the control unit 10, and irrigation liquid Y2 is supplied between the wound portion covering body 2 and the wound portion W as shown in FIG. 3, and the wound portion covering body 2 is bent and deformed upwardly convex (approximately dome-shaped) to fill the space between the wound portion covering body 2 and the wound portion W with irrigation liquid. At this time, the space S in FIG. 1 is filled with the irrigation liquid as shown by S2 in FIG. 3, the irrigation liquid also permeates the porous member 2a, and a positive pressure is applied to the wound portion covering body 2 in the direction X2. Thereby, it is possible to perform the irrigation (infection treatment) by immersing the surface of the wound portion W into the liquid to remove bacteria.

The irrigation liquid S2 held for a predetermined time by the control unit 10 is discharged by the irrigation liquid pump 6 and taken into the canister 8 as waste liquid. At this time, the number of bacteria in the discharged liquid is measured by the sensing unit 8a provided in the canister 8, and the number of irrigations by using the irrigation liquid pump 6 can be adjusted according to the measured number of bacteria. The supply and discharge of the irrigation liquid and the number of times thereof may be configured to be performed automatically by the control unit 10, or may be manually operated each time.

### <Modified embodiment 1>

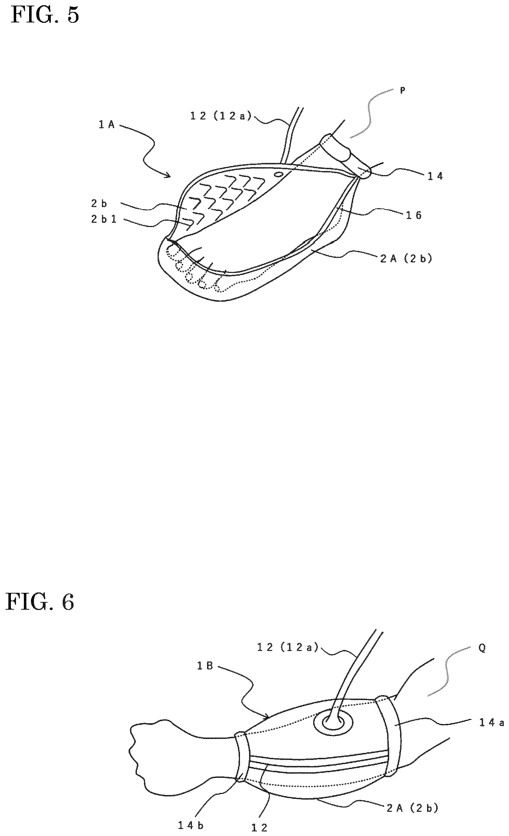

Next, a wound treatment device relating to the modified embodiment 2 will be described. FIG. 4 is a schematic diagram conceptually showing a wound treatment device 1A according to a modified embodiment of the wound treatment device 1 according to the above embodiment. FIG. 5 is a schematic diagram showing a wound portion covering body 2A of the wound treatment device 1A shown in FIG. 4 in an open state.

The wound treatment device 1A of this modified embodiment is characterized by providing a wound portion covering body 2A having an approximately shoe-shaped (foot cover-type) form that can cover the part of the foot below the ankle, and the other configurations are the same as those of the wound treatment device 1 of the above embodiment.

The wound portion covering body 2A has an opening that can be opened and closed by a watertight and airtight opening and closing mechanism 16, and when closed, as shown in FIG. 4, the ankle side portion is attached with a watertight and airtight waterproof tape (or band) 14. Examples of the opening and closing mechanism 16 include a waterproof zipper and Ziploc (registered trademark), and the like.

As shown in the open state in FIG. 5, in this modified embodiment, the inner porous member 2a laminated on the cover member 2b has a plurality of regularly arranged convex portions (projections) 2b1. Thereby, the porous member 2a can penetrate into the wound portion regardless of whether there is a wound on the instep, sole, front, or bottom of the foot, making it possible to perform the negative pressure wound therapy more reliably.

### <Modified embodiment 2>

Furthermore, a wound treatment device relating to another modified embodiment will be described. FIG. 6 is a schematic diagram conceptually showing a wound treatment device 1B according to the modified embodiment 2 of the wound treatment device 1 according to the above embodiment.

The wound treatment device 1B of this modified embodiment is characterized by providing a wound portion covering body 2B having an approximately cylindrical shape that can cover the entire periphery of the forearm (upper arm), and waterproof tapes (or bands) 14a, 14b are used in two places, and the other configurations are the same as those of the wound treatment device 1 of the above embodiment.

Representative embodiments and modifications of the present invention have been described above, but various further design modifications are possible, and all such design modifications are included within the technical scope of the present invention. For example, the pumping capacity of the negative pressure supply pump and the irrigation fluid pump can be selected appropriately depending on the location of the wound on which the wound treatment device is to be used, and the shape and dimensions of the wound portion covering body can also be selected appropriately depending on the shape and dimensions of the wound portion. Therefore, the wound portion may be in a three-dimensional or flat area, for example, the instep, sole, front, and bottom of the foot, calf, thigh, torso, forearm (upper arm), back of the hand, back, waist, abdomen, and the like.

Furthermore, in the above embodiments and modified embodiments, a case has been described in which the wound portion covering body 2 bends and deforms upwardly convex (approximately dome-shaped) and the irrigation liquid fills the space between the wound portion covering body 2 and the wound portion W, but as long as the effects of the present invention can be obtained, the wound portion covering body 2 may be in close contact with the wound portion to a certain extent.

In the above embodiments and modified embodiments, the wound treatment device includes the negative pressure supply pump and the irrigation liquid pump, but these negative pressure supply pump or irrigation liquid pump may be built into and integrated into the wound treatment device. Further, the negative pressure supply pump or the irrigation liquid pump may have a structure where the pump can be physically separable from the wound treatment device, or may be physically separated from the wound treatment device (separated each other) and may be incorporated and integrated into the wound treatment device or connected via tubes or the like when the wound treatment device is in use. In this way, various design modifications are possible for the structure of the wound treatment device, for example, from the viewpoint of cost, size, usability, and the like, and all of these design modifications are included within the technical scope of the present invention.

According to the wound treatment device of the present invention, it is possible to obtain a wound treatment device with which there is no increase in infection or delay in healing due to, inter alia, an irrigation liquid leakage or foam adjustment, embedment, or replacement, and that can effectively protect the surroundings of a wound portion to suppress complications and can be easily handled without requiring skill, and to provide a wound portion covering body used therein. That is, according to the wound treatment device and wound portion covering body used therein of the present invention, it is possible to realize infection control, early healing, prevention of complications, and shortened time and simplification, which were difficult to achieve with conventional wound treatments.

### EXPLANATION OF SYMBOLS

- 1, 1A, 1B: Wound treatment device
- 2, 2A, 2B: Wound portion covering body
- 2a: Porous member
- 2b: Cover member
- 4: Negative pressure supply pump
- 4a: Negative pressure supply pipe
- 6: Irrigation liquid pump
- 6a: Irrigation liquid pipe
- 8: Canister
- 8a: Sensing unit
- 10: Control unit
- 12: Pipe
- 12a: Pipe connection unit
- 14, 14a, 14b: Waterproof tape

## Claims

1. A wound treatment device **characterized by** comprising:
a wound portion covering body constituted from a flexible laminate including a porous member and a watertight cover member laminated on the porous member,
a negative pressure supply pump for supplying negative pressure to the porous member side,
an irrigation fluid pump for supplying an irrigation fluid to the porous member side and discharging the irrigation fluid from the porous member side,
a canister for intaking the discharged irrigation fluid, and
a control unit for controlling the negative pressure supply pump and the irrigation fluid pump,
wherein subjecting the wound portion covered on the porous member side by the wound portion covering body to negative pressure wound therapy and irrigation.

2. The wound treatment device according to claim 1, wherein the porous member has a number of convex portions on the surface opposite the cover member.

3. The wound treatment device according to claim 1 or 2, wherein the wound portion covering body is pad-shaped, approximately cylindrical, approximately bag-shaped or approximately shoe-shaped.

4. The wound treatment device according to claim 1 or 2, wherein the irrigation fluid is a liquid or a gas.

5. The wound treatment device according to claim 4, wherein the liquid contains an aqueous solution of peroxynitric acid.

6. The wound treatment device according to claim 1 or 2, wherein the canister is equipped with a sensing unit that measures the number of bacteria in the irrigation fluid.

7. The wound treatment device according to claim 1 or 2, wherein the control unit is capable of adjusting the number of washings depending on the number of bacteria.

8. The wound treatment device according to claim 1 or 2, **characterized in that** the negative pressure supply pump or the irrigation fluid pump is separable from the wound treatment device.

9. A wound portion covering body **characterized by**:
constituted from a flexible laminate including a porous member and a watertight cover member laminated on the porous member, and
used for subjecting the wound portion covered on the porous member side to negative pressure wound therapy and irrigation.

10. The wound portion covering body according to claim 9, wherein the porous member has a number of convex portions on the surface opposite the cover member.

11. The wound portion covering body according to claim 9 or 10, wherein the shape is pad-shaped, approximately cylindrical, approximately bag-shaped or approximately shoe-shaped.
